## Europäisches Patentamt

(19) **European Patent Office**

**Office européen des brevets**

(11) Publication number: **0 001 492**
**B1**

(12) **EUROPEAN PATENT SPECIFICATION**

(45) Date of publication of patent specification: **13.06.84**

(21) Application number: **78300423.7**

(22) Date of filing: **27.09.78**

(51) Int. Cl.³: **C 10 M 1/32, C 10 M 3/26, C 07 D 263/14**

(54) Certain oxazolines as load-carrying additives for gear oils.

(30) Priority: **03.10.77 US 838591**

(43) Date of publication of application:
**18.04.79 Bulletin 79/8**

(45) Publication of the grant of the patent:
**13.06.84 Bulletin 84/24**

(84) Designated Contracting States:
**BE DE FR GB NL SE**

(56) References cited:
**FR - A - 2 281 423**
**GB - A - 984 409**
**US - A - 2 759 894**
**US - A - 2 929 571**
**US - A - 4 035 309**

(73) Proprietor: **MOBIL OIL CORPORATION**
**150 East 42nd Street**
**New York New York 10017 (US)**

(72) Inventor: **Andress, Harry John Jr.1501 E. Elm Street**
**Wenonah New Yersey 08090**
**US (US)**

(74) Representative: **Jones, Alan John et al,**
**CARPMAELS & RANSFORD 43 Bloomsbury Square**
**London, WC1A 2RA (GB)**

**Description**

This invention relates to novel lubricant compositions containing certain oxazolines useful as load-carrying agents.

This invention provides a lubricant composition comprising a major amount of an oil of lubricating viscosity or a grease of lubricating viscosity and a minor amount of an oxazoline additive characterized in that the oxazoline additive is an alkenylsuccinic mono-oxazoline or an alkenylsuccinic bis-oxazoline in which the alkenyl group contains from 18—24 carbon atoms, and is present in a load-carrying amount.

U.S. Patent Specification No. 4,035,309 discloses the use of zinc or other metal complexes of oxazoline compounds as sludge dispersants in lubricating oils. While the equation at columns 4 and 5 thereof discloses the preparation of a bis-oxazoline by the reaction of a substituted succinic anhydride with tris-hydroxy-methyl aminomethane, this reference does not teach the use of such bis-oxazolines per se as lubricant additives as is the case in the present invention. Instead, this reference makes it quite clear that it is essential the oxazoline product be used in the form of a zinc complex.

French Patent Specification No. 2,281,423 is directed to a very broad group of oxazoline derivatives of alkenylsuccinic compounds in which the alkenyl groups contain from 8 to 300 carbon atoms, preferably 20 to 300 carbon atoms, and the use of these compounds as lubricant detergents. In Example 1 of this reference, the succinic anhydride used contains about 100 carbon atoms, and in Examples 2 and 3 the succinic anhydride used contains an average of 130 carbon atoms. There is no disclosure that the products of these Examples, which have longer alkenyl chains that those of additives used in the lubricant compositions of the present invention are useful as load-carrying additives.

The oxazoline employed in the present invention is an alkenylsuccinyl mono- or bis-oxazoline. The alkenylsuccinyl mono- or bis-oxazoline is made by reacting one mole of an alkenylsuccinic acid having 18 carbon atoms to 24 carbon atoms per alkenyl group with 1 or 2 moles of tris(hydroxymethyl)-aminomethane. The reaction is carried out at a temperature between 200°C and 250°C, preferably using an aromatic hydrocarbon, such as benzene or toluene, to remove water by azeotropic distillation through a water take-off trap, e.g. a Dean-Stark trap.

The lubricant compositions of the present invention contain an amount of the aforementioned additives sufficient to improve load-carrying properties. Generally, for most applications, the additive is present in an amount from 0.1 to 5% by weight and preferably in an amount from 0.1 to 1% by weight. The lubricants may comprise any materials that normally exhibit insufficient antiwear properties. A field of specific applicability is the improvement of liquid hydrocarbon oils boiling within the range from 24°C (75°F) to 538°C (1000°F). The lubricant oils used may be of any suitable lubricating viscosity range from 6 centistokes (cs.), i.e. 45 SSU, at 38°C (100°F) to 1300 cs. (6000 SSU) at 38°C (100°F) and, preferably, from 7 cs. to 54 cs. (50 to 250 SSU) at 99°C (210°F). These oils having viscosity indexes from 70 to 95 are preferred. The average molecular weights of these oils may range from 250 to 800. In general, the lubricant may comprise any mineral or synthetic oil of lubricating viscosity.

In instances where synthetic oils, or synthetic oils employed as the vehicle for the grease, are desired in preference to mineral oils, or in combination therewith, various compounds of this type may be successfully utilized. Typical synthetic vehicles include polyisobutylene, polybutenes, hydrogenated polydecenes, polypropylene glycol, polyethylene glycol, trimethylol propane esters, neopentyl and pentaerythritol esters, di(2-ethyl hexyl)sebacate, di(2-ethyl hexyl)adipate, dibutyl phthalate, fluorocarbons, silanes, esters of phosphorus containing acids, liquid ureas, ferrocene derivatives, hydrogenated mineral oils, chain-type polyphenyls, siloxanes and silicones (polysiloxanes), alkyl-substituted diphenyl ethers typified by a butyl-substituted bis(p-phenoxy phenyl)ether, and phenoxy phenylethers.

The aforementioned additives may be incorporated as antiwear agents in grease compositions. Such oils can also include hydraulic oils, if so desired. When high temperature stability is not a requirement of the finished grease, mineral oils having a viscosity of at least 4 cs. (40 SSU) at 66°C (150°F), and particularly those falling within the range from 10 cs. to 1300 cs. (60 SSu to 6000 SSU) at 38°C (100°F) may be employed. The lubricating vehicles of the improved greases of the present invention, containing the above-described additives, are combined with a grease-forming quantity of a thickening agent. For this purpose, a wide variety of materials may be dispersed in the lubricating vehicle in grease-forming quantities in such degree as to impart to the resulting grease composition the desired consistency. Exemplary of the thickening agents that may be employed in the grease formulation are non-soap thickeners, such as surface-modified clays and silicas, aryl ureas, calcium complexes and similar materials. In general grease thickeners may be employed which do not melt and dissolve when used at the required temperature within a particular environment; however, in all other respects, any material which is normally employed for thickening or gelling hydrocarbon fluids or forming greases can be used in preparing the improved greases.

The following examples illustrate the preparation of oxazoline additives used in lubricant compositions of the present invention.

Example 1

A mixture of 185 grams (0.5 mol) isooctadecenyl succinic acid and 121 grams (1.0 mol) tris-hydroxymethylamino methane was refluxed in toluene at a temperature of about 250°C until evolution of water ceased. The product is an isooctadecenyl succinyl bis-oxazoline.

Example 2

A mixture of 900 grams (1.0 mol) of $C_{18-24}$ dimer alkenylsuccinic anhydride and 121 grams (1.0 mol) tris-hydroxymethylamino methane was refluxed in toluene at a temperature of about 240°C until evolution of water ceased. The product is a $C_{18-24}$ dimer alkenylsuccinyl mono-oxazoline.

The Timken Load Test

Compositions according to the present invention were tested in the Timken Load Test. This test is a known test used to determine the load carrying properties of additives in lubricating oil compositions. The test is conducted by placing a steel test cup on a shaft which can be rotated at 800 rpm. Just below the cup in contact with it is a small stationary steel block. A load is placed on these parts by means of a lever arm which pushes the block upwards against the rotating cup, which acts as a roller bearing, while the lubricant flows between the two surfaces. The load is gradually increased at 10-minute intervals until failure occurs. Failure is determined by visual inspection during the running period.

The additives were blended in a conventionally refined mineral oil containing a sulfurized hydrocarbon, metal passivator, pour point depressant, antioxidant, emulsifier, antirust agent and defoamant. The results of the Timken OK Load Test are as follows:

| Additive | Conc. Wt. % | Load at failure in kg. (lbs.) |
|---|---|---|
| Base oil blend (Comparative Example) | 0 | 22·7 (50) |
| Base oil blend +Example 1 | ·0·1 | 27·2 (60) |
| Base oil blend +Example 2 | 0·1 | 27·2 (60) |

**Claims**

1. A lubricant composition comprising a major amount of an oil of lubricating viscosity or a grease of lubricating viscosity and a minor amount of an oxazoline additive characterized in that the oxazoline additive is an alkenylsuccinic mono-oxazoline or an alkenylsuccinic bis-oxazoline in which the alkenyl group contains from 18—24 carbon atoms, and is present in a load-carrying amount.

2. The composition of Claim 1 wherein the additive is an isooctadecenylsuccinyl bis-oxazoline.

**Revendications**

1. Une composition lubrifiante comprenant une quantité majeure d'une huile ayant une viscosité convenant à la lubrification ou d'une graisse ayant une viscosité convenant à la lubrification et une quantité mineure d'un additif à base d'oxazoline, caractérisée en ce que l'additif à base d'oxazoline est une alkénylsuccinyl mono-oxazoline ou une alkénylsuccinyl bis-oxazoline dans laquelle le groupe alkényle contient de 18 à 24 atomes de carbone ed ledit additif étant présent en une proportion conférant le caractère de résistance à la charge.

2. La composition selon la revendication 1, dans laquelle l'additif consiste en un isooctadécényl-succinyl-bis-oxazoline.

**Patentansprüche**

1. Eine Schmiermittel-Zusammensetzung mit einer Hauptmenge eines Öls einer schmierenden Viskosität oder eines Schmierfetts einer schmierenden Viskosität sowie einer geringeren Menge eines Oxazolin-Zusatzes, dadurch gekennzeichnet, daß der Oxazolin-Zusatz ein Alkenylbernsteinsäure-monooxazolin oder ein Alkenylbernsteinsäurebis-oxazolin ist, bei dem die Alkenylgruppe von 18—24 Kohlenstoffatomen enthält, und in einer lastaufnehmenden Menge vorhanden ist.

2. Zusammensetzung nach Anspruch 1, dadurch gekennzeichnet, daß der Zusatz ein Isooctadecenylbernsteinsäurebis-oxazolin ist.